# EUROPÄISCHE PATENTSCHRIFT

(11) **EP 2 436 415 B1**
(45) Veröffentlichungstag und Bekanntmachung des Hinweises auf die Patenterteilung: **12.08.2015**
(21) Anmeldenummer: 11007953.0
(22) Anmeldetag: 30.09.2011
(51) Int. Cl.: A61M 15/00, A61M 15/08, B05B 11/00

(54) **Austragvorrichtung zum Austrag von Flüssigkeiten**
Discharge device for discharging liquids
Dispositif de sortie destiné à la sortie de liquides

(30) Priorität: 30.09.2010 DE 102010047847
(43) Veröffentlichungstag der Anmeldung: 04.04.2012
(73) Patentinhaber: Aptar Radolfzell GmbH, 78315 Radolfzell (DE)
(72) Erfinder: Kohnle, Jörg, 78056 VS-Schwenningen (DE); Carrico, Silvio, 78224 Singen (DE); Helmlinger, Michael, 78215 Radolfzell (DE)
(74) Vertreter: Klement, Lukas

(56) Entgegenhaltungen:
- EP-A2- 1 125 637
- WO-A1-2006/095194

## Beschreibung

Die Erfindung betrifft eine Austragvorrichtung zum Austrag von Flüssigkeiten, insbesondere zum Austrag von pharmazeutischen Flüssigkeiten, mit einem Gehäuse, einer Steuerelektronik, einem Flüssigkeitsspeicher zur Speicherung der Flüssigkeit vor dem Austrag, einer Austragöffnung, einer manuell mittels einer ersten Betätigungshandhabe betätigbaren Fördereinrichtung, mittels derer die Flüssigkeit zum Zweck des Austrags vom Flüssigkeitsspeicher zur Austragöffnung gefördert werden kann, sowie mit einer Sperreinrichtung die einen Sperrzustand, in dem eine Betätigung der Fördereinrichtung mechanisch blockiert wird, und einen Freigabezustand, in dem die Betätigung der Fördereinrichtung möglich ist, einnehmen kann, um nach einem Austragvorgang für einen Sperrzeitraum einen weiteren Austragvorgang zu unterbinden. Dabei umfasst die Sperreinrichtung einer gattungsgemäßen Austragvorrichtung einen Sperrriegel, der gegenüber dem Gehäuse zwischen einer ersten und einer zweiten Stellung bewegbar ist, wobei die Sperreinrichtung derart ausgebildet ist, dass durch eine Bewegung des Sperrriegels aus der ersten Stellung in die zweite Stellung eine Überführung der Sperreinrichtung in den Freigabezustand verursacht wird. Dabei ist der Sperrriegel durch eine Federeinrichtung in Richtung der zweiten Stellung federkraftbeaufschlagt. Weiterhin ist eine erste Halteeinrichtung vorgesehen, mittels derer der Sperrriegel gegen die Kraft dieser Federeinrichtung in der ersten Stellung gehalten werden kann und die durch ein elektrisches Signal der Steuerelektronik lösbar ist.

Siehe als nächsten Stand der Technik WO 2006/095 194.

Eine gattungsgemäße Austragvorrichtung ist aus der DE 10 2008 064 559 A1 bekannt. Bei dieser ist vorgesehen, dass die mechanische Arbeit zur Überführung der Sperreinrichtung zwischen dem Sperrzustand und dem Freigabezustand durch den Benutzer während der Betätigung im Zuge eines Austragvorgangs in das System, eingebracht wird. Nach dem Austragvorgang ist diese mechanische Arbeit in einer oder mehreren Federeinrichtungen gespeichert, wobei die Federeinrichtung durch eine Halteeinrichtung im gespannten Zustand gehalten wird. Diese Halteeinrichtung wird bei den Ausführungsformen der DE 10 2008 064 559 A1 durch einen Permanentmagneten und einen Elektromagneten gebildet, wobei der Permanentmagnet die eigentliche Haltefunktion übernimmt und zur Überführung der Sperreinrichtung in den Freigabezustand mittels Elektromagneten kurzzeitig wirkungslos gemacht wird, so dass der Freigabezustand erzielt wird. Durch die Ansteuerung des Elektromagneten erfolgt also das Lösen der Halteeinrichtung.

Der Sperrriegel einer gattungsgemäßen Austragvorrichtung braucht nicht unmittelbar der Blockierung der Fördereinrichtung zu dienen. So sind bezogen auf die DE 10 2008 064 559 A1 sowohl der Stößel 78 als auch der Riegel 142 Sperrriegel im Sinne der vorliegenden Erfindung, da ihre Verlagerung jeweils mittelbar oder unmittelbar die Erreichung des Freigabezustand der Sperreinrichtung bewirkt.

Die Realisierung der Halteeinrichtung über einen Permanentmagneten und einen Elektromagneten stellt eine besonders vorteilhafte Lösung dar, führt jedoch auch zu dem Problem, dass starke Kraftstöße, die versehentlich oder mutwillig verursacht wurden, eine ungewollte Überführung der Sperreinrichtung in den Freigabezustand bewirken können, bevor der Sperrzeitraum abgelaufen ist. Hierdurch droht eine Überdosierung des im Flüssigkeitsspeicher gespeicherten Wirkstoffes.

### Aufgabe und Lösung

Aufgabe der Erfindung ist es, eine gattungsgemäße Austragvorrichtung hinsichtlich ihrer Sicherheit gegen Überdosierung zu verbessern.

Erfindungsgemäß ist hierzu eine zweite Halteeinrichtung vorgesehen, mittels derer der Sperrriegel gegen die Kraft der Federeinrichtung in der ersten Stellung gehalten werden kann und die durch eine manuelle Betätigung lösbar ist.

Eine erfindungsgemäße Austragvorrichtung weist somit neben der von der Steuerelektronik lösbaren ersten Halteeinrichtung eine zweite Halteeinrichtung auf, die bestimmungsgemäß durch den Benutzer der Austragvorrichtung bedient wird. Hierzu kann beispielsweise eine zweite Betätigungshandhabe an der Austragvorrichtung vorgesehen sein. Die zweite Halteeinrichtung kann aufgrund der Tatsache, dass sie manuell gelöst wird, robuster als die erste Halteeinrichtung ausgebildet sein, insbesondere dergestalt, dass ein Kraftstoß nicht mehr zur Verlagerung des Sperrriegels führt. Insbesondere kann die zweite Halteeinrichtung den Sperrriegel formschlüssig in seiner ersten Stellung halten, beispielsweise durch ein zusätzliches Halteglied, welches bei Nichtvorliegen einer manuellen Betätigung der zweiten Halteeinrichtung durch den Benutzer der Überführung des Sperrriegels aus dessen erster Stellung in die zweite Stellung im Wege steht.

Zur Überführung des Sperrriegels in die zweite Stellung müssen beide Halteeinrichtungen gelöst werden, die erste Halteeinrichtung mittels des elektrischen Signals und die zweite Halteeinrichtung mittels der manuellen Einflussnahme durch den Benutzer.

Eine erfindungsgemäße Austragvorrichtung weist vorzugsweise eine erste Halteeinrichtung mit einem Permanentmagneten auf, wobei der Permanentmagnet derart angeordnet und ausgebildet ist, dass durch ihn der Sperrriegel in der ersten Stellung gehalten werden kann. Diese Ausgestaltung mit einem Permanentmagneten hat sich als besonders zweckmäßig herausgestellt, da alleine die Überführung des Sperrriegels in die erste Stellung bereits zur Herstellung des Haltezustandes führt, ohne dass die erste Halteeinrichtung weitere mechanisch verlagerbare Bauteile aufweisen muss. Ebenfalls wird es als bevorzugt angesehen, wenn die erste Halteeinrichtung einen Elektromagneten umfasst, der bei ausreichender Bestromung durch die Steuerelektronik eine Kraftbeaufschlagung des Sperrriegels bewirkt, durch die eine Haltekraft des Permanentmagneten überwunden wird und damit ein Lösen der ersten Halteeinrichtung bewirkt wird.

Bevorzugt ist weiterhin, wenn ein mit der Steuerelektronik verbundener Sensor zur Erfassung des Lösens der zweiten Halteeinrichtung vorgesehen ist, wobei die Steuerelektronik dafür ausgebildet ist, in Reaktion auf das erfasste Lösen der zweiten Halteeinrichtung die erste Halteeinrichtung zu lösen, sofern der Sperrzeitraum bereits abgelaufen ist.

Bei dieser Weiterbildung wird somit nach Ablauf des Sperrzeitraums kein permanentes Lösen der ersten Halteeinrichtung, beispielsweise durch permanente Bestromung des Elektromagneten, bewirkt, da dies je nach Ausgestaltung der ersten Halteeinrichtung einen hohen Energiebedarf erfordern könnte. Stattdessen wird nach Ablauf des Sperrzeitraums die Steuerelektronik in einen Zustand versetzt, in dem sie beim Erkennen des manuell bewirkten Lösens der zweiten Halteeinrichtung auch die erste Halteeinrichtung löst. Der entsprechende Sensor kann an einer Betätigungshandhabe der zweiten Halteeinrichtung ebenso angeordnet sein, wie an dem oben genannten Halteglied, welches die Verlagerung des Sperrriegels in die zweite Stellung verhindert.

Da bei einer solchen Gestaltung, bei der nach Ablauf des Sperrzeitraums kein dauerhaftes Lösen der ersten Halteeinrichtung bewirkt wird, für einen Benutzer nicht ohne weiteres ersichtlich ist, ob der Sperrzeitraum bereits abgelaufen ist, wird es als bevorzugt angesehen, wenn eine mit der Steuerelektronik verbundene Anzeigeeinrichtung vorgesehen ist, wobei die Steuerelektronik dafür ausgebildet ist, den Ablauf des Sperrzeitraums mittels der Anzeigeeinrichtung zu signalisieren. Die Anzeigeeinrichtung kann für akustische und/oder optische Signalisierungen ausgebildet sein. Bevorzugt ist es, dass ein LC-Display oder ein vergleichbares Display Verwendung findet, auf welchem nach Ablauf des Sperrzeitraums ein entsprechendes Symbol, beispielsweise ein Schloss, dargestellt wird oder ausgeblendet wird. Besonders bevorzugt ist es, wenn drei verschiedene Darstellungsvarianten auf dem Display vorgesehen sind, die die drei Zustände der Austragvorrichtung "verriegelt, nicht entriegelbar", "verriegelt und entriegelbar" sowie "entriegelt und verwendungsbereit" voneinander unterscheidbar machen. Dies kann beispielsweise durch ein Symbol auf dem Display erzielt werden, welches in Abhängigkeit des Zustandes der Austragvorrichtung angeschaltet ist, blinkt oder ausgeschaltet ist. Der Benutzer kann somit mit einem Blick auf das Display den derzeitigen Zustand erkennen. Insbesondere kann auch eine Fehlfunktion des Gerätes, die einen Wechsel zwischen zwei Zuständen verhindert, leicht erkannt werden.

Bevorzugt ist es, wenn die zweite Halteeinrichtung ein mittels einer zweiten Betätigungshandhabe gegenüber dem Gehäuse bewegbares Halteglied aufweist, welches in einer Halteposition die Bewegung des Sperrgliedes mechanisch unterbindet und welches mittels der zweiten Betätigungshandhabe aus dieser Halteposition heraus bewegt werden kann. Bei einer besonders einfachen Ausgestaltung handelt es sich bei der Betätigungshandhabe und dem Halteglied um ein einstückiges Bauteil, welches translativ oder schwenkbeweglich am Gehäuse gelagert ist. Vorzugsweise ist das Halteglied in die Halteposition federvorgespannt, so dass es nach dem manuellen Lösen der zweiten Halteeinrichtung selbsttätig wieder die Halteposition einnimmt.

Bei einer besonders vorteilhaften Gestaltung ist weiterhin eine Dämpfungseinrichtung vorgesehen, welche die Bewegung zwischen dem Halteglied und dem Gehäuse verzögert. Hierdurch wird erreicht, dass auch ein besonders großer Kraftstoß nicht gleichzeitig zum Lösen der ersten Halteeinrichtung und der zweiten Halteeinrichtung führt, da die Dämpfungseinrichtung eine schnelle Verlagerung des Haltegliedes verhindert. Vorzugsweise kann die Dämpfungseinrichtung als elastisch verformbares Bauteil vorgesehen sein, beispielsweise als Schaumstoffelement, welches bei einer Verlagerung des Haltegliedes verformt wird. Die hierfür notwendige Energie ist über die zweite Betätigungshandhabe leicht einzubringen, während ein kurzzeitiger Kraftstoß, wie beim Aufschlagen einer Austragvorrichtung auf einem Fußboden, zur Verlagerung des Haltegliedes nicht ausreicht.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches anhand der Figuren erläutert wird. Dabei zeigen:
- Figur 1: eine erfindungsgemäße Austragvorrichtung in einer Gesamtdarstellung bei abgenommenem Außenmantel und
- Figur 2 bis Figur 5: in jeweils zwei Darstellungen einen Verwendungszyklus der Austragvorrichtung der Figur 1 ausgehend von einem Sperrzustand über die Herstellung des Freigabezustandes bis zur Widerherstellung des Sperrzustandes nach erfolgtem Austragvorgang.

### Detaillierte Beschreibung des Ausführungsbeispiels

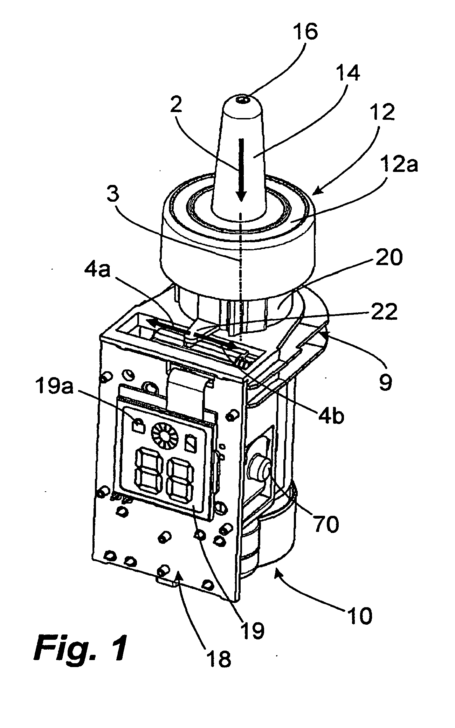

Figur 1 zeigt eine erfindungsgemäße Austragvorrichtung, bei der zur Verdeutlichung des Aufbaus ein Außenmantel eines Gehäuses 9 abgenommen wurde.

Die Austragvorrichtung verfügt über einen Flüssigkeitsspeicher 10 und einen Applikator 12 mit einer Nasenolive 14. Der Applikator 12 ist über eine Betätigungshandhabe 12a in Richtung des Pfeils 2 gegenüber dem Flüssigkeitsspeicher 10 hinabdrückbar, um hierdurch eine nicht dargestellte innen liegende Fördereinrichtung in Form einer Kolbenpumpe zu betätigen. Im Zuge einer solchen Betätigung der Fördereinrichtung wird pharmazeutische Flüssigkeit, beispielsweise ein Schmerzmittel, aus dem Flüssigkeitsspeicher 10 zu einer Austragöffnung 16 am distalen Ende der Nasenolive 14 gefördert und dort als Sprühstrahl ausgebracht.

Zur phasenweisen Unterbindung eines Austragvorgangs ist ein Sperrring 20 vorgesehen, der um eine in Richtung der Betätigungsrichtung 2 ausgerichtete Achse 3 drehbar angebracht ist und der in Abhängigkeit seiner Drehstellung mittels nicht näher dargestellter Anschlagsflächen ein Niederdrücken des Applikators 12 verhindert. An dem Sperrring 20 ist ein radial nach außen weisender Fortsatz 22 vorgesehen, der in nachfolgend noch detailliert erläuterter Weise zum Zwecke des Drehens des Sperrrings 20 verlagert wird.

Nach vorne wird die Austragvorrichtung durch eine elektronische Steuerelektronik 18 abgeschlossen, die ein LC-Display 19 zum Anzeigen von Statusinformationen aufweist.

Diese Steuerelektronik ist dafür ausgebildet, den Sperrring 20 am Ende eines Sperrzeitraums, innerhalb dessen ein Austragvorgang nicht erfolgen darf, in Richtung des Pfeils 4a mittels des Fortsatzes 22 in seine Freigabestellung zu bewegen bzw. diese Bewegung zu initiieren. Wenn der Sperrring sich in dieser Freigabestellung befindet, kann in oben genannter Art und Weise durch Hinabdrücken des Applikators 12 ein Austragvorgang bewirkt werden. Mit der Durchführung dieses Austragvorgangs erfolgt eine automatische Rückdrehung des Sperrrings 20 in Richtung des Pfeils 4b, einhergehend mit dem Beginn eines neuen Sperrzeitraums. Erst wenn dieser abgelaufen ist, wird durch die Steuerelektronik 18 wieder der Freigabezustand der Austragvorrichtung hergestellt.

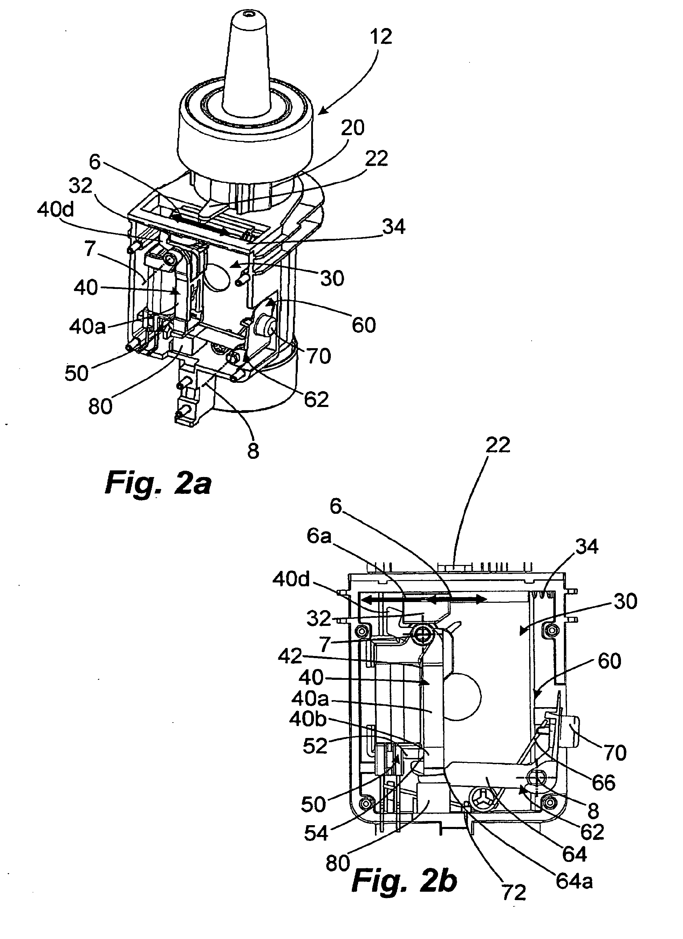

Die Figuren 2 bis 5 verdeutlichen die Bauelemente zum Halten des Sperrzustandes und ihre Zusammenwirken. Hierzu zeigen die Figuren 2 bis 5 jeweils einerseits die Austragvorrichtung als ganzes mit abgenommener Steuerelektronik 18 und andererseits eine isolierte Darstellung der erfindungswesentlichen Komponenten einer Sperreinrichtung 30 der Austragvorrichtung.

Anhand der Figuren 2a und 2b werden die wesentlichen Komponenten erläutert. Der bereits genannte Fortsatz 22 am Sperrring 20 wird durch einen Schlitten 32 geführt, welcher linear in Richtung des Pfeils 6 beweglich ist und welcher mittels einer Feder 34 permanent in jene Richtung 6a kraftbeaufschlagt wird, in die er zum Zwecke der Herstellung des Freigabezustandes der Austragvorrichtung bewegt werden muss. Die Bewegung des Schlittens 32 in Richtung 6a wird jedoch mittels eines Sperrriegels 40 und dessen Haltefortsatz 40d zeitweise unterbunden. Dieser Sperrriegel 40 ist um eine Achse 7 schwenkbar am Gehäuse angelenkt und wird bezogen auf die Perspektive der Figuren mittels einer Feder 42 permanent entgegen dem Uhrzeigersinn momentenbeaufschlagt. Dass diese Momentenbeaufschlagung durch die Feder 42 nicht unmittelbar zum Verschwenken des Sperrriegels 40 gegen den Uhrzeigersinn und damit zu einer Freigabe des Schlittens 32 und dessen Verlagerung in Richtung 6a führt, liegt an zwei Halteeinrichtungen 50, 60, die ein Verschwenken des Sperrriegels 40 verhindern können.

Die erste Halteeinrichtung 50 wird durch einen Permanentmagneten 52 gebildet, zu dem ein Hauptabschnitt 40a des Sperrriegels 40 einen Klappanker darstellt. Das freie Ende 40b des Sperrriegels 40 liegt daher durch die Kraft dieses Permanentmagneten gehalten im Sperrzustand der Sperreinrichtung 30 an einer zum Gehäuse 9 ortsfesten Anlagefläche 54 des Permanentmagneten 52 an. Die Magnetkraft des Permanentmagneten 52 der ersten Halteeinrichtung 50 reicht in einem Ruhezustand für sich alleine bereits aus, um ein Verschwenken des Sperrriegels 40 aus der in den Figuren 2a und 2b dargestellten Stellung entgegen dem Uhrzeigersinn zu verhindern.

Da jedoch im Falle eines starken Kraftstoßes, beispielsweise in Folge eines Herunterfallens der Austragvorrichtung, die magnetische Haltekraft überwunden werden könnte, ist zusätzlich die zweite Halteeinrichtung 60 vorgesehen. Diese zweite Halteeinrichtung 60 verfügt über ein um eine Schwenkachse 8 schwenkbares Halteglied 62 mit einem Halteabschnitt 64, der durch eine Anlagefläche 64a ebenfalls das Verschwenken des Sperrriegels 40 gegen den Uhrzeigersinn verhindert. Durch eine Feder 66 wird dieses Halteglied permanent in Richtung des Uhrzeigersinns momentenbeaufschlagt und daher sicher in seiner in den Figur 2a und 2b dargestellten Halteposition gehalten. Das Halteglied 62 ist drehfest verbunden mit einer zweiten Betätigungshandhabe 70, die in der in Figur 1 dargestellten Weise durch eine Aussparung des Gehäuses hindurch von außen zugänglich ist. Mittels dieser Betätigungshandhabe 70 ist es möglich, das Halteglied 62 und damit auch den Halteabschnitt 64 und die Anlagefläche 64a gegen die Kraft der Feder 66 gegen den Uhrzeigersinn zu verschwenken.

Die Figuren 2a und 2b zeigen, wie bereits erwähnt, den Sperrzustand der Sperreinrichtung 30. Sowohl durch die erste Halteeinrichtung 50 als auch durch die zweite Halteeinrichtung 60 wird in diesem Sperrzustand der Sperrriegel 40 gegen die Kraft der Feder 42 in Position gehalten, so dass er mittels des Haltefortsatzes 40d eine Verlagerung des Schlittens 32 in Richtung 6a und damit eine Überführung der Sperreinrichtung 30 in den Freigabezustand verhindert.

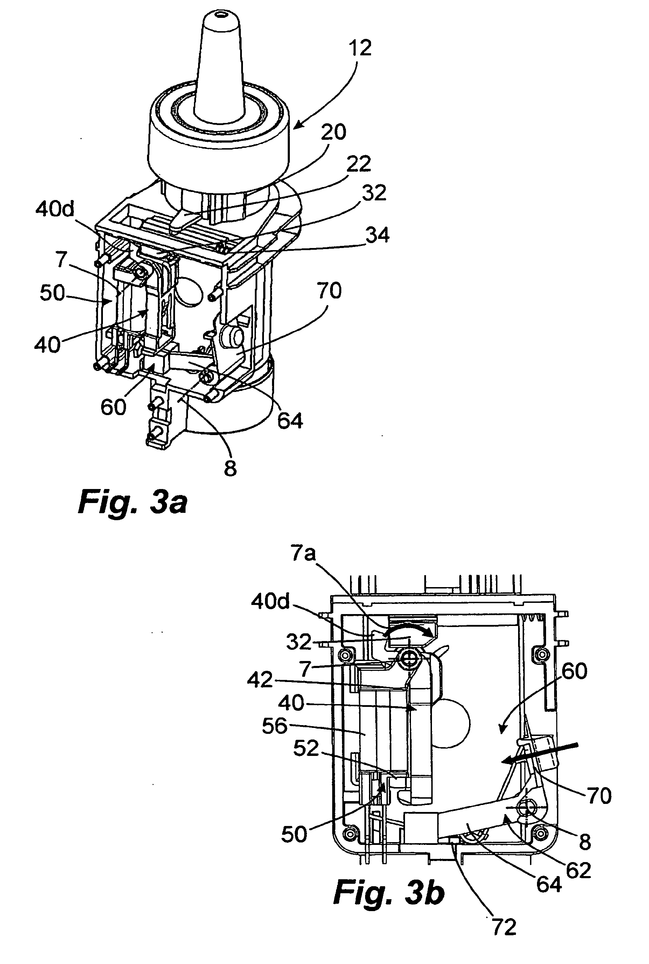

Eine isolierte manuelle Betätigung der Betätigungshandhabe 70 führt in diesem Zustand zwar zu einem Verschwenken des Halteabschnitts 64 im Uhrzeigersinn, wie in den Figuren 3a und 3b dargestellt ist. Hierdurch wird jedoch lediglich die zweite Halteeinrichtung 60 gelöst, während die erste Halteeinrichtung 50 auf Basis des Permanentmagneten 52 den Sperrriegel 40 zunächst unverändert in seiner ersten Stellung hält und somit einen Freigabezustand der Austragvorrichtung verhindert. Allerdings wird die Betätigung der Betätigungshandhabe 70 mittels eines Sensors 72 erfasst und an die Steuerelektronik 18 weitergegeben. Innerhalb des Sperrzeitraums führt diese Erfassung jedoch zu keinerlei durch die Steuerelektronik verursachten Einfluss auf die erste Halteeinrichtung 50.

Sobald ein Sperrzeitraum, innerhalb dessen bestimmungsgemäß kein Austragvorgang möglich sein soll, abgelaufen ist, wird dies auf dem LC-Display 19 verdeutlicht, indem ein zuvor konstant dargestelltes Symbol 19a in einen Blinkzustand übergeht. Der Benutzer erfährt hierdurch, dass die Austragvorrichtung aus einem verriegelten und nicht entriegelbaren Zustand in einen zwar immer noch verriegelten, jedoch entriegelbaren Zustand übergegangen ist. Wenn nach dem so signalisierten Ablauf des Sperrzeitraums mittels der Betätigungshandhabe 70 in der in den Figuren 3a und 3b dargestellten Weise das Halteglied 62 mit dem Halteabschnitt 64 verschwenkt wird, wird dies vom Sensor 72 erfasst und aufgrund der Tatsache, dass der Sperrzeitraum bereits abgelaufen ist, wird durch die Steuerelektronik 18 zusätzlich zur zweiten Halteeinrichtung auch die ersten Halteeinrichtung 50 gelöst. Hierfür wird ein Elektromagnet 56 bestromt. Dieser Elektromagnet 56 kompensiert die Haltekraft des Permanentmagneten 52, so dass in Reaktion das Moment der Feder 42 ausreicht, um den Sperrriegel 40 um die Achse 7 in Richtung des Pfeils 7a zu verschwenken.

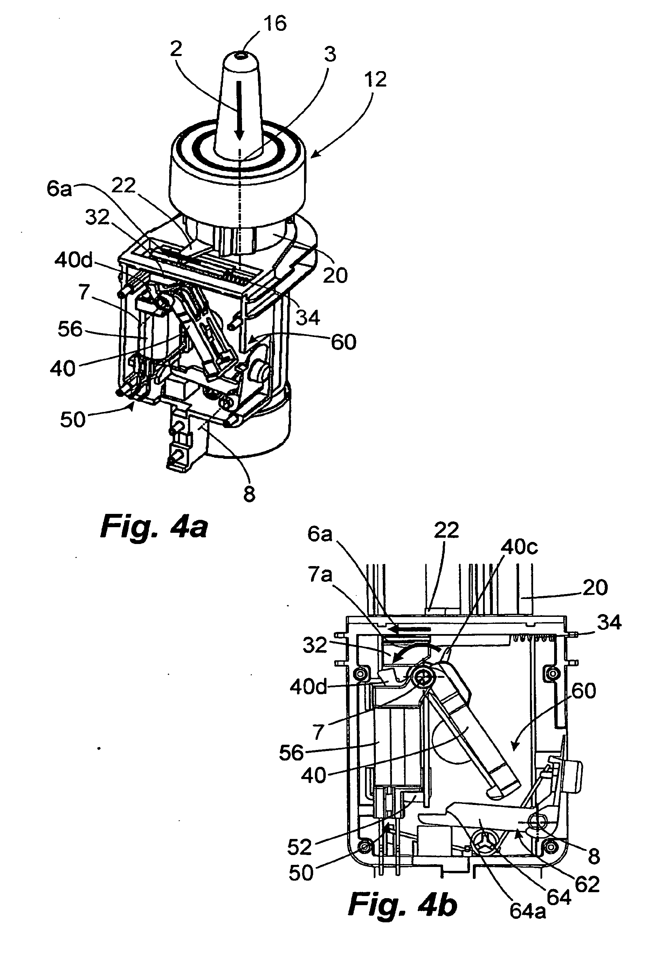

Die Figuren 4a und 4b verdeutlicht dieses Verschwenken des Sperrriegels 40 gemeinsam mit dem Haltefortsatz 40d. Dieses Verschwenken hat zur Folge, dass, angetrieben durch die Feder 34, der Schlitten 32 in Richtung des Pfeils 6a verlagert wird und damit der Sperrring 20 so um die Achse 3 verschwenkt wird, dass er ein Niederdrücken des Applikators 12 nicht länger verhindert. Somit ist ein entriegelter Zustand hergestellt worden. Dieser wird auf dem Display 19 dadurch dargestellt, dass das Symbol 19a nicht länger blinkt, sondern vollständig ausgebildet wird. Solange der entriegelte Zustand der Fig. 4a und 4b besteht, ist es dem Benutzer somit möglich, durch Niederdrücken des Applikators 12 die nicht dargestellte Fördereinrichtung zu betätigen und die pharmazeutische Flüssigkeit durch die Austragöffnung 16 hindurch auszutragen.

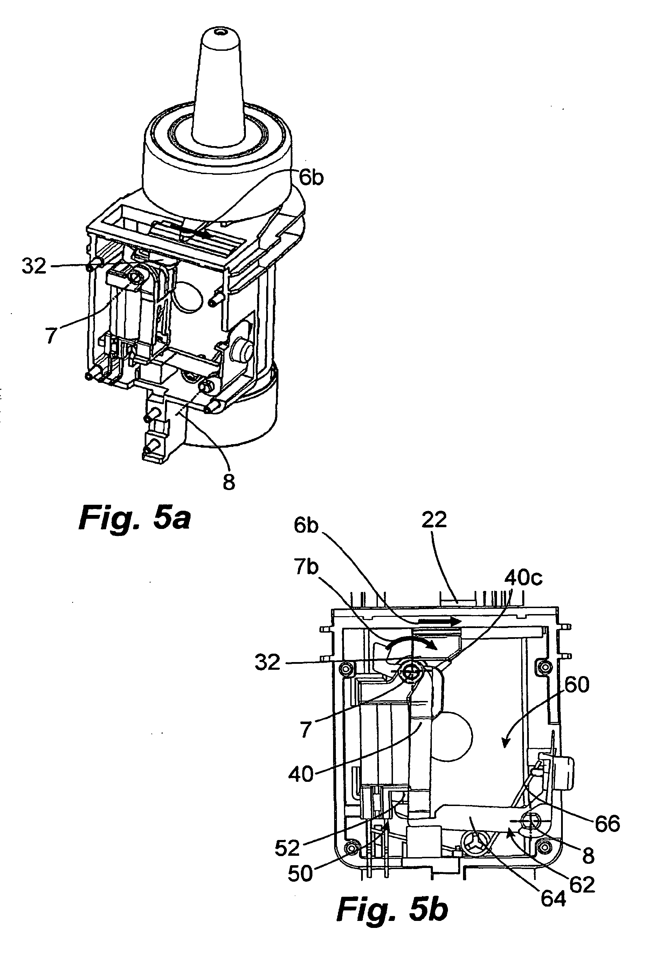

Während des Niederdrückens des Applikators 12 im Zuge dieses Austragvorgangs und/oder während des nachfolgenden Rückhubs des Applikators 12 wird durch eine vorliegend nicht dargestellte, jedoch aus der DE 10 2008 064 559 A1 bekannte Kulissenführung der Schlitten 32 in Richtung des Pfeils 6b zurückverlagert, wobei er dabei über einen Rückführabschnitt 40c des Sperrriegels 40 diesen entgegen der Federkraft der Feder 42 um die Achse 7 in Richtung des Pfeils 7b verschwenkt. Hierdurch gelangt der Sperrriegel 40 wieder in seine in den Figuren 2 und 3 dargestellte Position, wie sich auch aus den Figuren 5a und 5b ergibt. Die erste Halteeinrichtung 50 mit dem Permanentmagneten 52 hält der Sperrriegel 40 in dieser hierdurch wieder erreichten ersten Stellung ebenso wie der Halteabschnitt 64, welcher bei der Überführung des Sperrriegels aus dessen zweiter Stellung der Figuren 4a und 4b in die erste Stellung der Figuren 5a und 5b kurzfristig gegen die Kraft der Feder 66 ausgelenkt wird, um dann wieder mit der Anlagefläche 64a am Sperrriegel 40 zum Anliegen zu kommen.

Damit ist der Sperrzustand wieder erreicht und ein weiterer Sperrzeitraum beginnt, an dessen Ende in der beschriebenen Weise durch den Benutzer wieder der Freigabezustand hergestellt werden kann. Während der Sperrzustand besteht, wird dies durch das wieder konstant eingeblendete Symbol 19a auf dem Display 19 verdeutlicht.

Die dargestellte Austragvorrichtung gewährleistet auf besonders gute Weise die Aufrechterhaltung des Sperrzustands, während dessen kein Austragvorgang möglich ist. Durch die Verwendung der zwei Halteeinrichtungen 50, 60 ist die Gefahr stark vermindert, dass dieser Sperrzustand alleine durch eine Erschütterung und vor Ablauf des Sperrzeitraums aufgehoben wird. Zwar kann eine solche Erschütterung kurzzeitig möglicherweise die Haltekraft des Permanentmagneten 52 überschreiten. Die Halteeinrichtung 60 wird aufgrund ihrer Federvorspannung bei einer solchen Erschütterung jedoch nicht gelöst, so dass nach Ende der Erschütterung wieder beide Halteeinrichtungen 50, 60 den Sperrzustand aufrechterhalten. Zur Gewährleistung der Unempfindlichkeit der zweiten Halteeinrichtung 60 gegen Erschütterung ist bei der dargestellten Ausführungsform ein als Bremse wirkender Schaumgummi-Block 80 vorgesehen, der eine Auslenkung des Haltegliedes 62 durch eine manuelle Betätigung der Betätigungshandhabe 70 gestattet, einer durch einen kurzfristigen Kraftstoß verursachten Auslenkung des Haltegliedes 62 jedoch entgegensteht.

Wie die Darstellung der Figuren 5a und 5b verdeutlicht, wird der Schlitten 32 bei seiner Bewegung in Richtung des Pfeils 6b weit über seine Position im Sperrzustand der Sperreinrichtung 30, dargestellt in Figur 2, hinaus in Richtung des Pfeils 6b verlagert, wobei dies mit der oben beschriebenen Ausgestaltung der Kulissenführung zusammenhängt und in der DE 10 2008 064 559 A1 erläutert ist. Um zu verhindern, dass bei dauerhaft eingedrückter Betätigungshandhabe 70 die sich an den Zustand der Figur 5 anschließende Rückschlagbewegung des Schlittens 32 in Richtung des Pfeils 6a zu einem sofortigen Überwinden der Haltekraft der ersten Halteeinrichtung 50 führt, kann eine weitere Dämpfung bzw. Bremse vorgesehen sein, beispielsweise am Schlitten 32 selbst oder auch an der Lagerung des Sperrriegels 40.

## Patentansprüche

1. Austragvorrichtung zum Austrag von Flüssigkeiten, insbesondere zum Austrag von pharmazeutischen Flüssigkeiten, mit
- einem Gehäuse (9),
- einer Steuerelektronik (18),
- einem Flüssigkeitsspeicher (10) zur Speicherung der Flüssigkeit vor dem Austrag,
- einer Austragöffnung (16),
- einer manuell mittels einer ersten Betätigungshandhabe (12a) betätigbaren Fördereinrichtung, mittels derer die Flüssigkeit zum Zwecke des Austrags vom Flüssigkeitsspeicher (10) zur Austragöffnung gefördert werden kann, und
- einer Sperreinrichtung (30), die einen Sperrzustand, in dem eine Betätigung der Fördereinrichtung mechanisch blockiert wird, und einen Freigabezustand, in dem die Betätigung der Fördereinrichtung möglich ist, einnehmen kann, um nach einem Austragvorgang für einen Sperrzeitraum einen weiteren Austragvorgang zu unterbinden,
wobei
die Sperreinrichtung (30) einen Sperrriegel (40) umfasst, der gegenüber dem Gehäuse (9) zwischen einer ersten und einer zweiten Stellung bewegbar ist, wobei die Sperreinrichtung (30) derart ausgebildet ist, dass durch eine Bewegung des Sperrriegels (40) aus der ersten Stellung in die zweite Stellung eine Überführung der Sperreinrichtung (30) in den Freigabezustand erzielt wird und wobei
- der Sperrriegel (40) durch eine Federeinrichtung (42) in Richtung der zweiten Stellung federkraftbeaufschlagt ist und
- eine erste Halteeinrichtung (50) vorgesehen ist, mittels derer der Sperrriegel (40) gegen die Kraft der Federeinrichtung (42) in der ersten Stellung gehalten werden kann und die durch ein elektrisches Signal der Steuerelektronik (18) lösbar ist,
**dadurch gekennzeichnet, dass**
- eine zweite Halteeinrichtung (60) vorgesehen ist, mittels derer der Sperrriegel (40) gegen die Kraft der Federeinrichtung (42) in der ersten Stellung gehalten werden kann und die durch eine manuelle Betätigung lösbar ist.

2. Austragvorrichtung nach Anspruch 1,
**dadurch gekennzeichnet, dass**
die erste Halteeinrichtung (50) einen Permanentmagneten (52) aufweist, wobei der Permanentmagnet (52) derart angeordnet und ausgebildet ist, dass durch ihn der Sperrriegel (40) in der ersten Stellung gehalten werden kann.

3. Austragvorrichtung nach Anspruch 2,
**dadurch gekennzeichnet, dass**
die erste Halteeinrichtung (50) einen Elektromagneten (56) umfasst, der bei ausreichender Bestromung eine Kraftbeaufschlagung des Sperrriegels (40) bewirkt, durch die eine Haltekraft des Permanentmagneten (52) überwunden wird und damit ein Lösen der ersten Halteeinrichtung (50) bewirkt wird.

4. Austragvorrichtung nach einem der vorstehenden Ansprüche,
**dadurch gekennzeichnet, dass**
ein mit der Steuerelektronik (18) verbundener Sensor (72) zur Erfassung des Lösens der zweiten Halteeinrichtung (60) vorgesehen ist, wobei die Steuerelektronik (18) dafür ausgebildet ist, in Reaktion auf das erfasste Lösen der zweiten Halteeinrichtung (60) die erste Halteeinrichtung (50) zu lösen, sofern der Sperrzeitraum bereits abgelaufen ist.

5. Austragvorrichtung nach Anspruch 4,
**dadurch gekennzeichnet, dass**
eine mit der Steuerelektronik (18) verbundene Anzeigeeinrichtung (19) vorgesehen ist, wobei die Steuerelektronik (18) dafür ausgebildet ist, den Ablauf des Sperrzeitraums mittels der Anzeigeeinrichtung (19) anzuzeigen.

6. Austragvorrichtung nach einem der vorstehenden Ansprüche,
**dadurch gekennzeichnet, dass**
die zweite Halteeinrichtung (60) ein mittels einer zweiten Betätigungshandhabe (70) gegenüber dem Gehäuse bewegbares Halteglied (62) aufweist, welches in einer Halteposition die Bewegung des Sperrgliedes (40) mechanisch unterbindet und welches mittels der zweiten Betätigungshandhabe (70) aus dieser Halteposition herausbewegt werden kann.

7. Austragvorrichtung nach Anspruch 6,
**gekennzeichnet durch**
eine Dämpfungseinrichtung (80), welches die Bewegung zwischen dem Halteglied (62) und dem Gehäuse (9) verzögert, wobei das Halteglied (62) vorzugsweise als Schaumstoffelement (80) ausgebildet ist, welches bei einer Verlagerung des Haltegliedes (62) zusammengedrückt wird.

## Claims

1. A discharge device for discharging liquids, more particularly for discharging pharmaceutical liquids, comprising
- a housing (9),
- control electronics (18),
- a liquid storage receptacle (10) for storing the liquid prior to discharge thereof,
- a discharge orifice (16),
- a conveying device capable of being actuated manually by means of a first manual actuator (12a), by means of which the liquid can be transported to said discharge orifice from said storage receptacle (10) for the purpose of discharging the liquid, and
- a blocking device (30), which is capable of assuming a blocked state, in which actuation of said conveying device is mechanically blocked, and a released state, in which actuation of said conveying device is possible, in order to prevent another discharge operation from taking place during a blocking period following a discharge operation,
wherein
the blocking device (30) comprises an interlock device (40) that is capable of being moved relatively to the housing (9) between a first and a second position, said blocking device (30) being configured such that by moving said interlock device (40) from its first position to its second position a transfer of said blocking device (30) to the released state is achieved, and
wherein
- the interlock device (40) is spring biased by a spring device (42) in the direction of the second position and
- a first retaining device (50) is provided, by means of which said interlock device (40) can be retained in the first position against the force of said spring device (42) and which can be released by an electrical signal of said control electronics (18),
**characterized in that**
- a second retaining device (60) is provided, by means of which said interlock device (40) can be retained in the first position against the force of said spring device (42) and which can be released by manual actuation.

2. The discharge device according to claim 1, **characterized in that**
the first retaining device (50) comprises a permanent magnet (52), which permanent magnet (52) is disposed and configured such that it can retain said interlock device (40) in its first position.

3. The discharge device according to claim 2, **characterized in that**
the first retaining device (50) comprises an electromagnet (56) which, when sufficiently energized, applies force to said interlock device (40), by means of which a retaining force of said permanent magnet (52) is overcome so as to release said first retaining device (50).

4. The discharge device according to any one of the preceding claims, **characterized in that**
a sensor (72) connected to said control electronics (18) is provided for detection of the release of said second retaining device (60), the control electronics (18) being adapted to release said first retaining device (50) in response to the detected release of said second retaining device (60), provided that the blocking period has elapsed.

5. The discharge device according to claim 4, **characterized in that**
a display device (19) connected to said control electronics (18) is provided, the control electronics (18) being adapted to indicate the expiry of the blocking period by means of the display device (19).

6. The discharge device according to any one of the preceding claims, **characterized in that**
said second retaining device (60) comprises a retaining element (62) which is capable of being moved relatively to said housing by means of a second manual actuator (70) and which in a retaining position mechanically prevents movement of said blocking element (40) and which is capable of being moved out of its retaining position by means of said second manual actuator (70).

7. The discharge device according to claim 6, **characterized by**
a damping device (80) which retards the movement between the retaining element (62) and the housing (9), said retaining element (62) being preferably in the form of a foamed block (80) which is compressed when the retaining element (62) is displaced.

## Revendications

1. Dispositif de distribution pour la distribution de liquides, en particulier pour la distribution de liquides pharmaceutiques, comprenant
- un boîtier (9),
- une électronique de commande (18),
- un réservoir de liquide (10) pour stocker le liquide avant la distribution,
- une ouverture de distribution (16),
- un dispositif de transport pouvant être actionné manuellement au moyen d'une première manette d'actionnement (12a), au moyen duquel le liquide peut être transporté à partir du réservoir de liquide (10) jusqu'à l'ouverture de distribution à des fins de distribution, et
- un dispositif de blocage (30) qui peut adopter un état de blocage, dans lequel un actionnement du dispositif de transport est bloqué mécaniquement, et un état de libération, dans lequel l'actionnement du dispositif de transport est possible, afin d'empêcher une opération de distribution supplémentaire pendant une période de blocage après une opération de distribution,
le dispositif de blocage (30) comportant un verrou de blocage (40) qui peut être déplacé par rapport au boîtier (9) entre une première et une deuxième position, le dispositif de blocage (30) étant réalisé de telle sorte qu'un transfert du dispositif de blocage (30) dans l'état de libération soit obtenu par un déplacement du verrou de blocage (40) de la première position à la deuxième position et
- le verrou de blocage (40) étant sollicité par une force de ressort en direction de la deuxième position au moyen d'un dispositif de ressort (42) et
- un premier dispositif de maintien (50) étant prévu, au moyen duquel le verrou de blocage (40) peut être maintenu dans la première position à l'encontre de la force du dispositif de ressort (42) et lequel peut être libéré par un signal électrique de l'électronique de commande (18), **caractérisé en ce que**
- un deuxième dispositif de maintien (60) est prévu, au moyen duquel le verrou de blocage (40) peut être maintenu dans la première position à l'encontre de la force du dispositif de ressort (42) et lequel peut être libéré par un actionnement manuel.

2. Dispositif de distribution selon la revendication 1,
**caractérisé en ce que**
le premier dispositif de maintien (50) comprend un aimant permanent (52), l'aimant permanent (52) étant disposé et réalisé de telle sorte qu'au moyen de celui-ci, le verrou de blocage (40) puisse être maintenu dans la première position.

3. Dispositif de distribution selon la revendication 2,
**caractérisé en ce que**
le premier dispositif de maintien (50) comporte un électroaimant (56), lequel provoque une application de force du verrou de blocage (40) en cas d'alimentation en courant électrique suffisante, au moyen de laquelle application de force une force de maintien de l'aimant permanent (52) est surmontée et une libération du premier dispositif de maintien (50) est par conséquent provoquée.

4. Dispositif de distribution selon l'une quelconque des revendications précédentes,
**caractérisé en ce**
**qu'**un capteur (72) connecté à l'électronique de commande (18) est prévu pour la détection de la libération du deuxième dispositif de maintien (60), l'électronique de commande (18) étant réalisée pour libérer le premier dispositif de maintien (50) en réaction à la libération détectée du deuxième dispositif de maintien (60), pour autant que la période de blocage ait déjà expiré.

5. Dispositif de distribution selon la revendication 4,
**caractérisé en ce**
**qu'**un dispositif d'affichage (19) connecté à l'électronique de commande (18) est prévu, l'électronique de commande (18) étant réalisée pour afficher l'expiration de la période de blocage au moyen du dispositif d'affichage (19).

6. Dispositif de distribution selon l'une quelconque des revendications précédentes,
**caractérisé en ce que**
le deuxième dispositif de maintien (60) comprend un organe de maintien (62) pouvant être déplacé par rapport au boîtier au moyen d'une deuxième manette d'actionnement (70), lequel organe de maintien empêche mécaniquement le déplacement de l'organe de blocage (40) dans une position de maintien et lequel peut être déplacé hors de cette position de maintien au moyen de la deuxième manette d'actionnement (70).

7. Dispositif de distribution selon la revendication 6,
**caractérisé par**
un dispositif d'amortissement (80), lequel ralentit le déplacement entre l'organe de maintien (62) et le boîtier (9), l'organe de maintien (62) étant réalisé de préférence en tant qu'élément en mousse (80), lequel est comprimé dans le cas d'un déplacement de l'organe de maintien (62).
